# EUROPÄISCHE PATENTSCHRIFT

(11) **EP 0 515 799 B1**
(45) Veröffentlichungstag und Bekanntmachung des Hinweises auf die Patenterteilung: **13.04.1994**
(21) Anmeldenummer: 92105680.0
(22) Anmeldetag: 02.04.1992
(51) Int. Cl.: B01J 10/00, B01J 19/24, B01F 3/04, C07C 69/76

(54) **Reaktor für phasenheterogene Reaktionen**
Reactor for phase-heterogeneous reactions
Réacteur pour des réactions aux phases hétérogènes

(30) Priorität: 29.05.1991 DE 4117592
(43) Veröffentlichungstag der Anmeldung: 02.12.1992
(73) Patentinhaber: HÜLS AKTIENGESELLSCHAFT, 45764 Marl (DE)
(72) Erfinder: Piotrowski, Bernhard, Dr., W-5204 Lohmar 1 (DE); Korte, Hermann-Josef, Dr., W-5216 Niederkassel 2 (DE)

(56) Entgegenhaltungen:
- DE-B- 1 264 413
- FR-A- 1 512 557
- GB-A- 1 580 177
- US-A- 4 342 876

## Beschreibung

### Technisches Gebiet

Die Erfindung betrifft einen Reaktor zur Durchführung phasenheterogener Reaktionen, bei dem in horizontaler Ebene in Teilbereichen des Reaktorquerschnitts eine im wesentlichen vertikal nach oben gerichtete und in anderen Teilbereichen eine im wesentlichen vertikal nach unten gerichtete Strömung des Reaktionsmediums stattfindet und wobei der Reaktor im wesentlichen nur in den Teilbereichen mit nach oben gerichteter Strömung Mittel zur Dispergierung der diskontinuierlichen (dispersen) Phase aufweist.

Bei phasenheterogenen Reaktionen, insbesondere von Gas-Flüssig-Reaktionen mit kontinuierlicher flüssiger Phase und disperser Gasphase, sind häufig die für die Reaktion notwendigen Stoff- und/oder Wärmetransportprozesse durch die Phasengrenzfläche entscheidend für den Verlauf der Reaktion und für die Ausbeute des Reaktors. Vor allem für exotherme Reaktionen mit kontinuierlicher flüssiger und disperser gasförmiger Phase muß eine Konglomeration der Gasblasen vermieden werden.

Die vorliegende Erfindung betrifft insbesondere einen Reaktor zur Durchführung der stark exothermen Oxidationsreaktion von p-Xylol und p-Toluylsäuremonomethylester mit Luft beim sogenannten Witten-DMT-Verfahren (DE-C3-28 05 915, DE-C2-37 04 720, WO-90/09 367). Charakteristisch für diese Reaktion ist es, daß mit dem Sauerstoff ein hoher Teil an Inertgas durch den Reaktor geführt werden muß, wodurch eine unerwünschte Blasenkonglomeration gefördert wird.

### Stand der Technik

Aus Ullmann, Band 3, Seite 357 ff, sind diverse Grundtypen von Reaktoren für phasenheterogene Reaktionen bekannt.

Die Oxidation von p-Xylol und p-Toluylsäuremonomethylester mit Luft wird bei bestehenden Anlagen überwiegend in einfachen Blasensäulenreaktoren ohne Dispergiereinrichtungen durchgeführt, bei denen zentral im unteren Teil des Reaktors das sauerstoffhaltige Reaktionsgas eingeleitet wird. Die Blasen lagern sich nach kurzer Strömungslänge zum großen Teil zu Großblasenagglomeraten zusammen, die die Flüssigkeitszirkulation bestimmen. Die Wärmetauscher sind bei diesen Reaktoren horizontal in einem äußeren Ringraum in der Nähe der Behälterwandung angeordnet. Das flüssige Reaktionsmedium strömt entlang dieser Wärmetauscherrohre wieder nach unten und wird dort von den Blasen erneut mit nach oben gerissen (Mammutpumpenprinzip).

Durch das Auftreten von Großblasen in den Reaktoren zeigen sich folgende unerwünschte Erscheinungen:
- hohe Schaumbildung am Reaktorkopf,
- durch die in den Gasblasenzwischenräumen nicht abgeführte Reaktionswärme bedingter Ausbeuteverlust,
- hohe Zirkulationsgeschwindigkeiten der Flüssigkeit mit dadurch zum Teil bedingten Kurzschlußströmen.

Diese Nachteile wirken sich zum einen in der erzielbaren Oxidationsausbeute sowie in dem fluiddynamisch bedingten, zum Teil instabilen Verhalten der Reaktoren aus.

Aus der US-A-4,342,876 ist ein Schlaufenreaktor zur Durchführung der Oxidationsreaktion von p-Xylol und p-Toluylsäuremonomethylester mit Luft bekannt, bei dem in einem ersten Reaktorteil die flüssigen Reaktanden und die sauerstoffhaltige Luft mit dem umlaufenden Reaktionsmedium gemischt und zur Reaktion gebracht werden. In einem davon getrennten Reaktorteil wird das durch die Reaktion aufgeheizte Reaktionsmedium mit den darin enthaltenen flüssigen Produkten durch längsangeströmte Wärmetauscherrohre abgekühlt. Der Umlauf des Reaktionsmediums wird durch den Dichteunterschied der beiden Phasen aufrechterhalten.

Bei diesem Reaktor besteht die Gefahr, daß die in Form von Blasen in den Reaktor eingeleitete gasförmige Phase zu Großblasen konglomeriert, wodurch einerseits die Phasengrenzfläche und damit die Reaktionsgeschwindigkeit verringert wird und andererseits die zwischen den großen Gasblasen eingeschlossene flüssige Phase unzureichend durchmischt wird, wodurch hohe Temperaturspitzen und damit Ausbeuteverluste auftreten können.

Aus der EP-A1-0 075 742 ist ein gattungsgemäßer Schlaufenreaktor zur Durchführung einer Gas-Flüssigkeits-Reaktion bekannt, bei dem die gasförmige und die flüssige Phase am Boden des Reaktors in einen offenen Innenzylinder als Mischzone eingeführt werden. Die Mischzone ist mit nicht näher bezeichneten Füllkörpern oder Einbauten versehen. Hierdurch wird eine Redispergierung der Gasblasen erreicht. Bei dieser Reaktorart wird die Reaktionswärme durch Verdampfen von flüssigem Reaktionsmedium abgeführt. Ein derartiger Reaktor läßt sich für die Oxidationsreaktion von p-Xylol und p-Toluylsäuremonomethylester mit Luft nicht einsetzen.

### Aufgabe

Aufgabe der vorliegenden Erfindung war es daher, einen gattungsgemäßen Reaktor zur Verfügung zu stellen, der zur Durchführung der Oxidationsreaktion von p-Xylol und p-Toluylsäuremonomethylester mit Luft einsetzbar ist und bei dem das Auftreten von Temperaturspitzen vermieden wird.

Ein weiteres Anliegen der Erfindung ist es, die Ausbeute des Reaktors zu erhöhen. Schließlich ist es ein weiteres Anliegen der Erfindung, die Wärmeabfuhr bei exothermen Reaktionen zu verbessern, das Auftreten von Großblasen zu verringern und eine homogenere Temperaturverteilung innerhalb des Reaktors zu gewährleisten.

Schließlich soll mit der Erfindung eine kostengünstige Umrüstung bestehender Reaktoren ermöglicht werden.

### Darstellung der Erfindung

Die Erfindung löst diese Aufgabe bei einem gattungsgemäßen Reaktor durch die kennzeichnenden Merkmale des Anspruchs 1.

Im Gegensatz zu üblichen Schlaufenreaktoren ist der innere Bereich des Reaktors mit nach oben gerichteter Strömung dabei nicht von dem äußeren Reaktorringraum abgetrennt. Trotz der als (Re-)Dispergiereinrichtungen bevorzugt eingesetzten Dispergiersiebe mit geringem Durchtrittsquerschnitt von bevorzugt < 30 % werden die Blasen nicht in den äußeren Reaktorquerschnitt abgelenkt. Die Blasen treten vielmehr vollständig durch die Siebe und erneuern dabei ihre (Phasen-)Oberfläche.

Die Hauptströmung im Reaktor ist im Reaktorinneren im Bereich der Dispergiereinrichtungen durch die geringere Dichte der Blasen nach oben gerichtet, wird oben im Bereich des Flüssigkeitsspiegels nach außen umgelenkt und gelangt außen entlang der Behälterwandung (Ringraum) wieder nach unten. Hier erfolgt bevorzugt die Abkühlung des Reaktionsmediums, z.B. mit längsangeströmten Kühlrohren. Durch die erfindungsgemäß vorgesehene Querströmung von dem äußeren Reaktorringraum in die Reaktorzone mit aufwärts gerichteter Strömung, d.h. zu den Dispergiereinrichtungen, bewirkt eine Abkühlung des Reaktionsmediums an dieser Stelle und eine zusätzliche Vermischung der flüssigen Phase.

Ein besonderer Vorteil der Erfindung liegt darin, daß auch bestehende Blasensäulenreaktoren, wie sie gewöhnlich für die Reaktion von p-Xylol und p-Toluylsäuremonomethylester mit Luft beim Witten-DMT-Verfahren eingesetzt werden, kostengünstig entsprechend der Erfindung umgerüstet werden können, indem lediglich einige Dispergiersiebe in den Reaktor eingebaut werden.

Soweit nach einer bevorzugten Ausführung der Erfindung Dispergiersiebe als Mittel zur Dispergierung der leichteren Phase (Gasphase) eingesetzt werden, beträgt der mittlere Abstand der Dispergiersiebe untereinander bevorzugt das 0,5 - 1,5-fache ihres Durchmessers, wobei bei nicht kreisförmigen Sieben als Durchmesser der Durchmesser eines flächengleichen runden Siebes angesetzt wird.

Grundsätzlich können in den Reaktor Wärmetauscher sowohl im inneren Bereich mit nach oben gerichteter Strömung als auch im äußeren Bereich mit nach unten gerichteter Strömung angeordnet werden. Im allgemeinen sind die Wärmetauscher jedoch aus konstruktiven Gründen außen, d.h. in den Teilbereichen mit nach unten gerichteter Strömung angeordnet.

Von besonderem Vorteil ist es, wenn die disperse (diskontinuierliche) leichtere Phase im Bereich der Dispergiersiebe in den Reaktor eingeleitet wird. Hierzu wird vorteilhaft eine größere Anzahl vertikal in verschiedenen Ebenen übereinander angeordneter Luftdüsen aus einem vertikal im Reaktor angeordneten Zuleitungsrohr gespeist. Diese Einspeisung der dispersen Phase in verschiedenen Ebenen des Reaktors im Bereich der Dispergiersiebe hat besondere Vorteile:

Der Anteil der noch nicht abreagierten Reaktanden in der dispersen Phase (z.B. Sauerstoff) kann in vertikaler Richtung annähernd konstant eingestellt werden. Weiterhin unterstützen die frisch eingeleiteten Gasblasen eine Quervermischung der kontinuierlichen (flüssigen) Phase und sorgen damit für einen noch besseren Temperaturausgleich. Insbesondere die synergistische Wechselwirkung zwischen der Möglichkeit der freien Strömung von den Teilbereichen des Reaktors mit im wesentlichen nach unten gerichteter und den Teilbereichen mit im wesentlichen nach oben gerichteter Strömung und der bevorzugten Einleitung der dispersen (diskontinuierlichen) Phase in den Reaktor im Bereich der Dispergiersiebe führt zu einer erheblichen Steigerung der Ausbeute des Reaktors.

Nach einer weiteren vorteilhaften Ausgestaltung der Erfindung wird die Hauptströmung in dem Reaktor - im inneren Bereich nach oben und im äußeren Bereich nach unten gerichtet - z.B. mittels einer Pumpe zusätzlich unterstützt. Durch das Aufprägen einer im wesentlichen vertikal nach oben gerichteten zentralen Strömung der kontinuierlichen Phase wird der Umsatz und die Ausbeute des Reaktors weiter gesteigert.

Bevorzugt wird die ganz oder teilweise abreagierte leichtere Phase am Kopf und die schwerere Phase am Boden des Reaktors abgezogen.

### Kurze Beschreibung der Zeichnungen

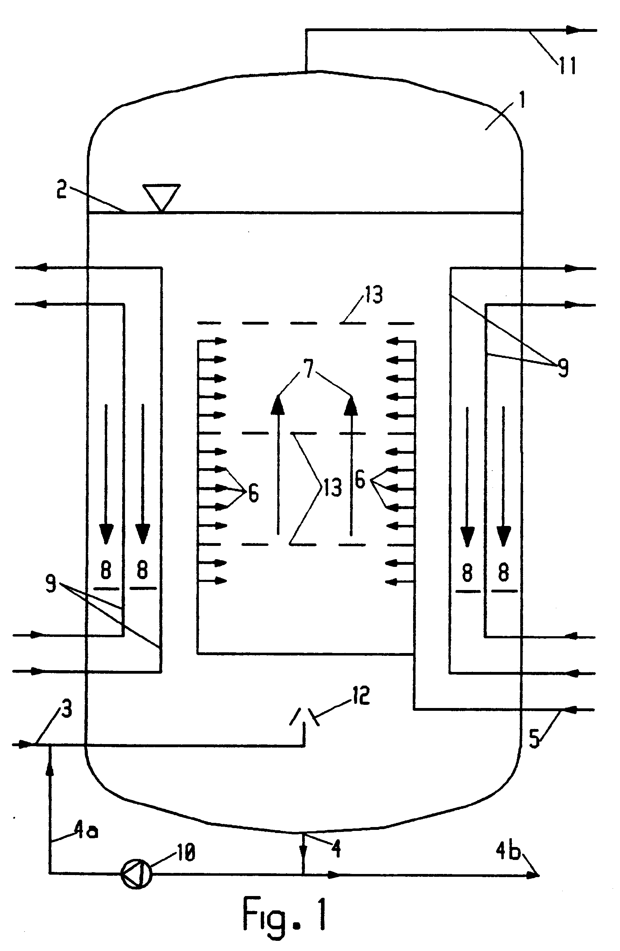

Im folgenden wird die Erfindung anhand eines Ausführungsbeispiels sowie der Zeichnung näher erläutert. Es zeigt dabei
Figur 1 einen erfindungsgemäßen Reaktor (schematisch).

### Bester Weg zur Ausführung der Erfindung

Der in Fig. 1 dargestellte Reaktor 1 dient zur Oxidation eines Gemisches aus para-Xylol (p-X) und para-Toluylsäure-methylester (p-TE) in der flüssigen Phase in Abwesenheit von Lösungsmitteln und von Halogenverbindungen bei einem Druck von etwa 7 bar und einer Temperatur von etwa 160 °C mit Luftsauerstoff in Gegenwart von gelösten Schwermetalloxidationskatalysatoren, z.B. in Gegenwart eines Gemisches von Kobalt- und Mangan-Verbindungen (vgl. DE-PS 20 10 137). Dabei entsteht ein Gemisch aus Monomethylterephthalat (MMT) und p-Toluylsäure (p-TA). Dieser Prozeß ist z.B. in der DE-A1 39 04 586 (WO-90/09 367) ausführlich beschrieben.

Die am Boden des Reaktors 1 abgezogene und bereits weitestgehend abreagierte schwere Oxidat-Phase 4 wird zu einem Teil über die Pumpe 10 als Umlaufmenge 4a in den unteren Teil des Reaktors 1 zurückgeführt. Der verbleibende Teilstrom 4b kann dagegen einer weiteren Reaktionsstufe, z.B. Oxidations- oder Veresterungsstufe, zugeleitet werden.

In die Umlaufmenge 4a werden die zur Oxidation erforderlichen Einsatzstoffe 3, wie z.B. p-Xylol, p-Toluylsäuremonomethylester und Katalysator, zugemischt. Um ein ungewolltes Austreten der Einsatzstoffe 3 mit der abreagierten Oxidat-Phase 4 durch Kurzschlußströmung zu vermeiden, werden die Einsatzstoffe 3 und die Umlaufmenge 4a so in den unteren Teil des Reaktors 1 eingeleitet, daß eine im wesentlichen vertikal nach oben gerichtete Strömung 7 aufgeprägt wird. Zur Verstärkung dieser Strömung 7 werden der Mengenstrom 3 und die Umlaufmenge 4a so in eine Vorrichtung, z.B. Flüssigkeitsstrahler 12, eingeleitet, daß ein Mehrfaches des eingesetzten Mengenstromes 3 und der Umlaufmenge 4a zur Aufprägung der Strömung 7 genutzt werden. Quer zu dieser Strömung 7 wird über die Lufteinleitstelle 5 die Luft als diskontinuierliche leichtere Phase mit Hilfe von Luftdüsen 6 in den Bereich der Dispergiersiebe 13 geleitet. Dabei wird die diskontinuierliche leichtere Phase von der Strömung 7 mitgerissen und beim Durchströmen der Dispergiersiebe 13 mehrfach aufgeteilt bzw. dispergiert. Die hierbei eintretende mehrfache Neubildung der Phasengrenzflächen zwischen der diskontinuierlichen und der kontinuierlichen Phase führt zu einem besonders intensiven und einheitlichen Stoff- und Wärmeaustausch der beiden Phasen.

Dieser Vorgang wird in vorteilhafter Weise, wie die in Fig. 1 dargestellt, an jedem Dispergiersieb 13 wiederholt, bis daß die diskontinuierliche leichtere Phase, weitestgehend vom Sauerstoff abreagiert, die Phasengrenzfläche 2 des Reaktionsmediums und den Reaktor am Kopf als leichtere Phase 11 verläßt. Die vertikal nach oben gerichtete Strömung 7 weicht nach dem Verlassen des letzten Dispergiersiebes 13 im Bereich der Phasengrenzfläche 2 in den äußeren Teilbereich des Reaktors nach unten aus und bildet so die nach unten gerichtete Strömung 8. Dabei wird die Strömung 8 an den in diesem Bereich angeordneten Rohren 9 des Wärmeaustauschers vorbeigeführt, wobei bevorzugt die Abkühlung des Reaktionsmediums in der Strömung 8 erfolgt. Teilmengen dieser Strömung 8 werden von der Luft (diskontinuierliche leichtere Phasen) von dem äußeren Reaktorringraum in die Reaktorkernzone mitgeführt, so daß eine zusätzliche Vermischung der leichteren Phase mit dem abgekühlten Reaktionsmedium aus der Strömung 8 erfolgt.

## Patentansprüche

1. Reaktor (1) zur Durchführung phasenheterogener Reaktionen, insbesondere von Gas-Flüssig-Reaktionen mit kontinuierlicher flüssiger und disperser (diskontinuierlicher) Gasphase,
- bei dem in horizontaler Ebene in Teilbereichen des Reaktorquerschnitts eine im wesentlichen vertikal nach oben gerichtete (7) und in anderen Teilbereichen eine im wesentlichen vertikal nach unten gerichtete Strömung (8) des Reaktionsmediums stattfindet
- und wobei der Reaktor (1) im wesentlichen nur in den Teilbereichen mit nach oben gerichteter Strömung (7) Mittel (13) zur Dispergierung der diskontinuierlich Phase aufweist,
***dadurch gekennzeichnet***, daß im Bereich der Dispergiermittel (13) eine freie Strömung des Reaktionsmediums von den Teilbereichen mit nach unten gerichteter Strömung (8) zu den Teilbereichen mit nach oben gerichteter Strömung (7) möglich ist.

2. Reaktor (1) nach Anspruch 1, ***dadurch gekennzeichnet***, daß als Mittel zur Dispergierung Dispergiersiebe (13) mit einer freien Durchtrittsfläche kleiner 30 % und mit einem mittleren Abstand zueinander vom 0,5 bis 1,5-fachen ihres Durchmessers eingesetzt werden.

3. Reaktor (1) nach einem der Ansprüche 1 oder 2, ***dadurch gekennzeichnet,*** daß in den Teilbereichen des Reaktors mit nach unten gerichteter Strömung (8) Wärmetauscher (9) angeordnet sind.

4. Reaktor (1) nach Anspruch 2, ***gekennzeichnet durch*** Mittel (6) zur Einleitung der diskontinuierlichen leichteren Phase in den Reaktor im Bereich der Dispergiersiebe (13).

5. Reaktor (1) nach einem der Ansprüche 1 bis 4, ***gekennzeichnet durch*** Mittel (10), (12) zum Aufprägen einer im wesentlichen vertikal nach oben gerichteten zentralen Strömung der kontinuierlichen Phase.

6. Reaktor (1) nach einem der Ansprüche 1 bis 5, ***dadurch gekennzeichnet***, daß die leichtere, ganz oder teilweise abreagierte Phase (11) am Kopf des Reaktors abgezogen wird.

7. Verwendung eines Reaktors nach einem der Ansprüche 1 bis 6 zur Oxidation von p-Xylol und p-Toluylsäuremonomethylester mit Luft.

## Claims

1. Reactor (1) for carrying out phase-heterogeneous reactions, in particular gas-fluid-reactions with continuous fluid and dispersed (discontinuous) gas phase,
- in which there is a flow (7) of the reaction medium which is directed substantially vertically upwards in a horizontal plane in partial regions of the reactor cross section, and there is a flow (8) of the reaction medium which is directed substantially vertically downwards in other partial regions,
- and wherein the reactor (1) has means (13) for dispersing the discontinuous phase substantially just in the partial regions with upwardly directed flow (7),
characterised in that, in the region of the dispersing means (13), a free flow of the reaction medium is possible from the partial regions with downwardly directed flow (8) to the partial regions with upwardly directed flow (7).

2. Reactor (1) according to claim 1, characterised in that dispersing sieves (13) are used as dispersing means, the sieves having a free penetration area of less than 30% and having an average spacing relative to each other of 0.5 to 1.5 times their diameter.

3. Reactor (1) according to one of the claims 1 or 2, characterised in that heat exchangers (9) are arranged in the partial regions of the reactor with downwardly directed flow (8).

4. Reactor (1) according to claim 2, characterised by means (6) for the introduction of the discontinuous, comparatively light phase into the reactor in the region of the dispersing sieves (13).

5. Reactor (1) according to one of the claims 1 to 4, characterised by means (10), (12) for impelling a central flow of the continuous phase, which flow is directed substantially vertically upwards.

6. Reactor (1) according to one of the claims 1 to 5, characterised in that the comparatively light phase (11), the reaction of which is completely or partly brought to completion, is drawn off at the top of the reactor.

7. Use of a reactor according to one of the claims 1 to 6 for the oxidation of p-xylene and p-toluic acid monomethyl ester with air.

## Revendications

1. Réacteur (1) pour effectuer des réactions à phases hétérogènes, notamment des réactions gaz-liquide à phase liquide continue et phase gazeuse dispersée (discontinue),
- dans lequel dans un plan horizontal, a lieu, dans des zones partielles de la section transversale du réacteur, un écoulement (7) du milieu de réaction dirigé sensiblement à la verticale vers le haut, et dans d'autres zones partielles, un écoulement (8) du milieu de réaction sensiblement dirigé à la verticale vers le bas,
- et le réacteur (1) ne comportant essentiellement que dans les zones partielles à écoulement (7) dirigé vers le haut, des moyens (13) pour la dispersion de la phase discontinue,
caractérisé en ce que dans la zone des moyens de dispersion (13) peut s'établir un écoulement libre du milieu de réaction, des zones partielles à écoulement (8) dirigé vers le bas vers les zones partielles à écoulement (7) dirigé vers le haut.

2. Réacteur (1) selon la revendication 1, caractérisé en ce que l'on utilise en guise de moyens pour la dispersion, des tamis de dispersion (13) présentant une surface de passage libre inférieure à 30% et un espacement moyen les uns par rapport aux autres compris entre 0,5 et 1,5 fois leur diamètre.

3. Réacteur (1) selon l'une des revendications 1 ou 2, caractérisé en ce que dans les zones partielles du réacteur à écoulement (8) dirigé vers le bas, sont disposés des échangeurs de chaleur (9).

4. Réacteur (1) selon la revendication 2, caractérisé par des moyens (6) destinés à introduire la phase plus légère discontinue dans le réacteur, dans la zone des tamis de dispersion (13).

5. Réacteur (1) selon l'une des revendications 1 à 4, caractérisé par des moyens (10), (12) pour communiquer un écoulement central dirigé sensiblement à la verticale vers le haut, à la phase continue.

6. Réacteur (1) selon l'une des revendications 1 à 5, caractérisé en ce que la phase (11) plus légère, ayant totalement ou partiellement réagi, est extraite à la tête du réacteur.

7. Utilisation d'un réacteur selon l'une des revendications 1 à 6 pour l'oxydation par l'air de p-xylol et p-monométhylester d'acide toluique.
